# EUROPEAN PATENT APPLICATION

(11) **EP 0 645 128 A1**
(43) Date of publication of application: **29.03.1995**
(21) Application number: 94114779.5
(22) Date of filing: 20.09.1994
(51) Int. Cl.: A61F 6/04

(54) **Condom**

(30) Priority: 24.09.1993 IT MI932052
(71) Applicant: Di Pietro, Antonino, I-20064 Gorgonzola, Milan (IT)
(72) Inventor: Di Pietro, Antonino, I-20064 Gorgonzola, Milan (IT)
(74) Representative: Luksch, Giorgio, Dr.-Ing.

(57) **Abstract**

There is disclosed a condom characterized in that it comprises two membranes, an external and an internal one, between which it is inserted at least one substance.

## Description

The present invention relates to a condom.

As it is well known in the art, condoms, among the means having a contraceptive function, have a remarkable importance since they are a protective barrier against the infections caused by viruses.

Condoms, as known, even guaranteeing a good impermeability and safety, show remarkable problems.

In fact, authoritative studies which have been done recently in the medical field, allowed to ascertain that the latex membrane of which conventional condoms are made, show porosities through which viruses, such as the HIV virus which is responsive of the AIDS infection, are able to pass easily, thus causing very dangerous infections.

Further, the very high influence of the membrane breakings which very frequently occur during the use of said condoms is known.

The aim of present invention is to solve the above said problems and drawbacks, making a condom which is able to offer an effective and suitable barrier against virus infections and which shows a very high contraceptive activity.
Another purpose of the present invention is to make a condom which is able to eliminate the troublesome irritations which may occur on skin using the conventional condoms during sexual intercourses.

These aims are achieved by a condom characterized in that it comprises two membranes, an external and an internal one, between which it is inserted at least one substance which is able to guarantee the maximum contraceptive safety, even if one of said membranes should break.

Besides, the condom of the present invention due to its feasible characteristic peculiarities, gives a high guaranty as to tolerability and safety and it is easily obtainable using elements and materials which are commonly available.

Therefore, such a condom is advantageous from the economical point of view too.

The aims and the purposes hereabove exposed, which will be better understood in the following disclosure, are obtained by a condom, according to the present invention, characterised in that it comprises two membranes, one internal and one external, between which at least one substance, such as, for instance, a lubricating, an antiseptic, virucidal and/or a contraceptive agent or a mixture thereof, is present.

Further peculiarities and advantages of the present invention will be better understood by an analysis of the description of a preferred, non limiting embodiment of the condom of the present invention, which is illustrative but non limitative of the present invention, through the attached drawings wherein:
- figure 1 represents a front and partially sectioned view of the structure of the present condom, having a double membrane, between which at least a substance is present;
- figure 2 represents a front view of an alternative embodiment of the same condom, wherein said two membranes can be linked together by suitable weldings, for instance made in the hollow space originated between them.

With reference to the numbers of the above figures, the present condom, indicated as a whole with reference number 1, is characterized in that it is made of a structure which comprises an external membrane 2 and an internal membrane 3.

Particularly, said membranes 2 and 3, which in the preferred non limiting embodiment have a thickness of about 0,03 mm, are linked together by welding on the ring 5.

The materials employed for carrying out the present invention may be whatever, depending on occasion, as long as they are compatible with the specific use; preferably gum latex is used. Obviously the variables of the carrying out of the present condom will vary depending on the material used.

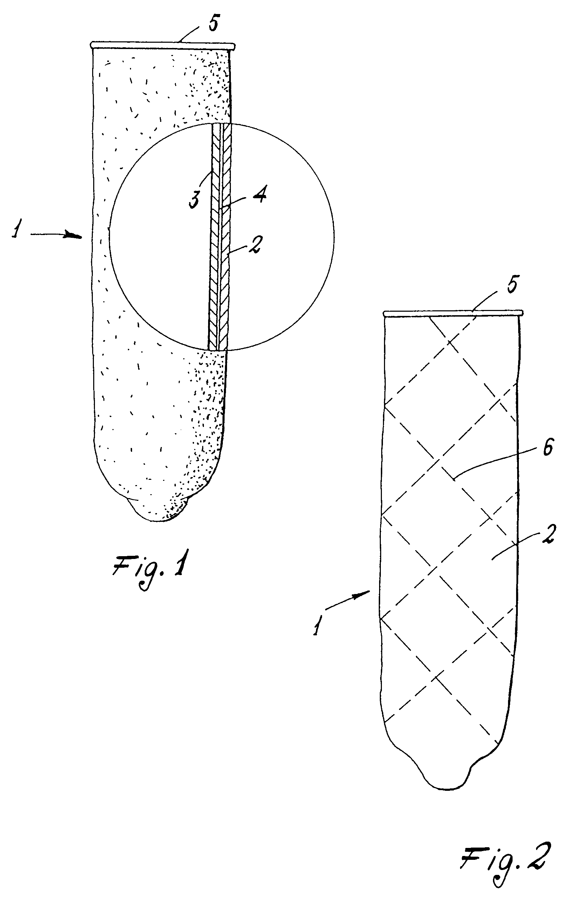

In another preferred embodiment, said membranes 2 and 3 can be linked between them by suitable weldings 6 carried out, for instance, in the hollow space 4 originated between said membranes, starting from the base to the top of the condom 1, as it can be seen in Figure 2.

At least one substance is inserted in said hollow space.

Such a substance may be a lubricating agent or a substance able to combat viruses, fungi and bacteria such as an antiseptic, contraceptive, virucidal agent or a mixture thereof.

The presence of a lubricating agent, in said hollow space 4, allows to check the friction between said membranes, or to greatly check the rubbing of the condom 1 on skin, thereby eliminating the painful irritations which may frequently occur during a sexual intercourse.

Before using it, the present condom is rolled up in a discoid shape while when it has to be used it is unrolled in such a way to get a shape basically identical to the one of conventional condoms.

The presence of a double membrane gives to the present condom the greatest contraceptive safety, even if the breaking or the perforation of one of the membranes would occur.

Further, the condom itself is able to guarantee on overall protection against virus infections, particularly against the HIV virus.

In fact, in one of the preferred embodiments of the present invention, if the passage of a virus throughout the porosities of one of said membranes would occur, it would be immediately eliminated by the liquid therein present, without allowing to said virus to pass over the barrier of the other of said membranes.

It will be appreciated from the foregoing that the above aims and purposes are achieved by the condom of the present invention.

Further it is to be noted the very good level of sensibility attained during sexual intercourses using the condom of the present invention, given that the sexual pleasure sensations provoked by pressor stimulations, do not undergo to any alterations.

The object of the present invention may be the subject of many changes and variations, all falling within the scope of the present inventive concept; each of which being replaceable with another feature which is technically equivalent.

## Claims

1. Condom (1) characterized in that it comprises two membranes, an external (2) and an internal (3) one, between which it is inserted at least one substance.

2. Condom (1) according to claim 1, characterized in that said substance is a lubricating agent or a substance able to combat viruses, fungi and bacteria such as an antiseptic, contraceptive, virucidal agent or a mixture thereof.

3. Condom (1) according to any of the previous claims, characterized in that said membranes (2,3) are linked together by welding themselves to the ring (5) apt to act as the base rim of the condom.

4. Condom (1) according to any of the previous claims, characterized in that said membranes (2,3) are made of gum latex and have a thickness of 0,03 mm.

5. Condom (1) according to any of the previous claims, characterized in that said membranes (2,3) are linked together by weldings (6) carried out in the hollow space (4) originated between them.
